# FASCICULE DE BREVET EUROPEEN

(11) **EP 2 665 513 B1**
(45) Date de publication et mention de la délivrance du brevet: **15.08.2018**
(21) Numéro de dépôt: 12704877.5
(22) Date de dépôt: 19.01.2012
(51) Int. Cl.: A61M 31/00, A61D 7/00

(54) **DISPOSITIF D'INTRODUCTION D'ÉLÉMENTS DANS UN CORPS**
VORRICHTUNG ZUR EINFÜHRUNG VON ELEMENTEN IN EINEN KÖRPER
DEVICE FOR INTRODUCING ELEMENTS INTO A BODY

(30) Priorité: 20.01.2011 FR 1150453
(43) Date de publication de la demande: 27.11.2013
(73) Titulaire: Ceva Santé Animale, 33500 Libourne Cedex (FR)
(72) Inventeur: LE GALL, Antoine, F-38120 Saint Egreve (FR); LEGAT, Jean-Jacques, F-38690 Colombe (FR)
(74) Mandataire: Vidon Brevets & Stratégie
(86) Numéro de dépôt international: PCT/FR2012/050115
(87) Numéro de publication internationale: WO 2012/098335

(56) Documents cités:
- EP-A2- 1 018 375
- US-A- 3 682 175
- US-A- 5 279 555

## Description

### Domaine technique

L'invention concerne de façon générale un dispositif d'introduction d'éléments dans un corps. L'invention s'applique plus particulièrement à l'introduction de médicament dans le corps d'animaux.

### Technique antérieure

Ce type de dispositifs est couramment utilisé, afin d'administrer des médicaments à des animaux ou afin d'implanter des traceurs dans des animaux. Ces médicaments ou traceur peuvent être introduits par la bouche ou par les voies vaginales. Le traceur comprend par exemple une puce électronique permettant l'identification de l'animal et/ou la gestion et le suivi automatique des déplacements de l'animal et/ou de contrôler son accès selon des zones autorisées ou non.

La publication GB 696 796 décrit un dispositif d'introduction d'un médicament à l'intérieur du corps d'un animal. Ce dispositif d'introduction comprend un tube de guidage couplé à une poignée fixe et raccordé à un tube réservoir débouchant dans le tube de guidage et prévu incliné au-dessus du tube d'alimentation. Une tige de poussée est prévue coulissante dans le tube de guidage pour, une fois le tube de guidage introduit dans l'animal, pousser le médicament dans l'animal. Cette tige de poussée est couplée à une poignée mobile, articulée par une liaison pivot à l'extrémité de la poignée fixe. La poignée mobile a une forme de L inversé, l'extrémité libre de la grande barre du L étant couplée à la tige de poussée, la liaison pivot étant prévue à la jonction de la grande barre du L, l'extrémité libre de la petite barre du L étant couplée à une extrémité d'un ressort hélicoïdal dont l'autre extrémité est couplée au tube de guidage au niveau de l'implantation de la poignée fixe. Le ressort hélicoïdal tend à rapprocher la petite barre du L de la poignée mobile du tube de guidage et donc à faire reculer la tige de poussée afin de pouvoir recharger le tube de guidage. L'inclinaison du tube réservoir par rapport au tube de guidage rend ce dispositif d'introduction encombrant et limite la longueur du médicament utilisable. De plus le ressort hélicoïdal est très exposé pendant l'utilisation. Il risque en effet facilement de s'accrocher dans les poils ou les crins de l'animal. Ce dispositif d'introduction est donc peu fiable. De plus, ce ressort est volumineux, difficile à nettoyer et il rend l'assemblage du dispositif d'introduction compliqué. Les publications US 6,024,728 et GB 651 524 décrivent des dispositifs d'introduction sensiblement similaires et comportant des ressorts hélicoïdaux disposés de manière semblable.

La publication US 5,588,452 décrit un dispositif d'introduction d'un médicament à l'intérieur du corps d'un animal, sensiblement similaire au précédent. Il s'en différencie par la position du ressort hélicoïdal prévue entre les extrémités des poignées fixe et mobile raccordées respectivement au tube de guidage et à la tige de poussée. La position de ce ressort implique une ouverture importante entre les poignées fixe et mobile. Le débattement nécessaire pour assurer l'introduction du médicament est donc important ce qui rend ce dispositif d'introduction difficile à utiliser.

La publication WO 2006/128 586 décrit un dispositif d'introduction, par la bouche d'un animal, d'un traceur. Ce dispositif d'introduction comprend un tube de guidage dont l'extrémité creuse est apte à recevoir le bol et couplé à une poignée fixe. Le tube de guidage comprend deux sections angulairement distinctes, l'une apte à appuyer contre le coin de la gueule de l'animal, l'extrémité de l'autre étant apte à atteindre la région du pharynx. Une tige de poussée est prévue coulissante dans le tube de guidage pour, une fois le tube de guidage introduit dans l'animal, pousser le traceur dans l'animal. Cette tige de poussée est couplée à une poignée mobile, articulée à l'extrémité de la poignée fixe par une liaison pivot. Les poignées fixe et mobile sont séparées par une lame ressort en V, dont la pointe est située au niveau de la liaison pivot, et tendant à écarter les poignées l'une de l'autre. Cette lame ressort est encombrante et rend l'assemblage de ce dispositif d'introduction compliqué. Les publications FR 2 469 175, GB 524 916 et GB 1 412 312 décrivent des dispositifs sensiblement similaires comportant également des lames ressort en V.

La publication EP 1 018 375 A2 divulgue un dispositif ayant une languette venant de matière avec une gâchette.

### Exposé de l'invention

Le but de l'invention est de remédier à ces inconvénients en proposant un dispositif d'introduction d'un élément dans un corps, de conception et d'assemblage simples, efficace, fiable et facile à utiliser.

A cet effet, l'invention a pour objet un dispositif d'introduction d'éléments dans un corps, selon la revendication 1. Le dispositif d'introduction selon l'invention est donc de conception simple, il comporte un nombre de pièces à assembler limité rendant son montage facile. La configuration particulière de l'élément de rappel élastique le rend de plus facile à utiliser, fiable et efficace.

Le dispositif d'introduction selon l'invention peut avantageusement présenter une ou plusieurs des particularités suivantes :
- l'une au moins des première, seconde liaisons pivot comporte au moins un tenon et au moins une zone de guidage du tenon prévus respectivement l'un sur la poignée mobile, l'autre sur l'une des tige de poussée, poignée fixe, et définissant respectivement avec chacune une pièce monobloc ;
- la languette ressort comporte au moins deux portions angulairement distinctes définissant entre-elles au moins un coude, l'une des poignée fixe, poignée mobile comportant au moins un bossage apte à recevoir le coude pour bloquer la position de la languette ressort par rapport respectivement à la poignée fixe, poignée mobile ;
- la poignée fixe et la poignée mobile comportent respectivement des formes complémentaires aptes à venir en butée entre-elles pour délimiter une position ouverte maximale ;
- la poignée mobile est couplée à la tige de poussée, la poignée fixe est formée par le prolongement direct de la paroi du tube de guidage et comporte deux flancs reliés par une paroi dorsale délimitant un logement, entièrement ouvert frontalement, apte à recevoir la poignée mobile au moins dans sa position fermée.

### Présentation sommaire des dessins

La présente invention sera mieux comprise et d'autres avantages apparaîtront à la lecture de la description détaillée d'un mode de réalisation pris à titre d'exemple nullement limitatif et illustré par les dessins annexés, dans lesquels :
- la figure 1 est une vue en perspective du dispositif d'introduction selon l'invention ;
- la figure 2 est une vue en coupe partielle selon son plan médian du dispositif d'introduction de la figure 1.

### Description des modes de réalisation

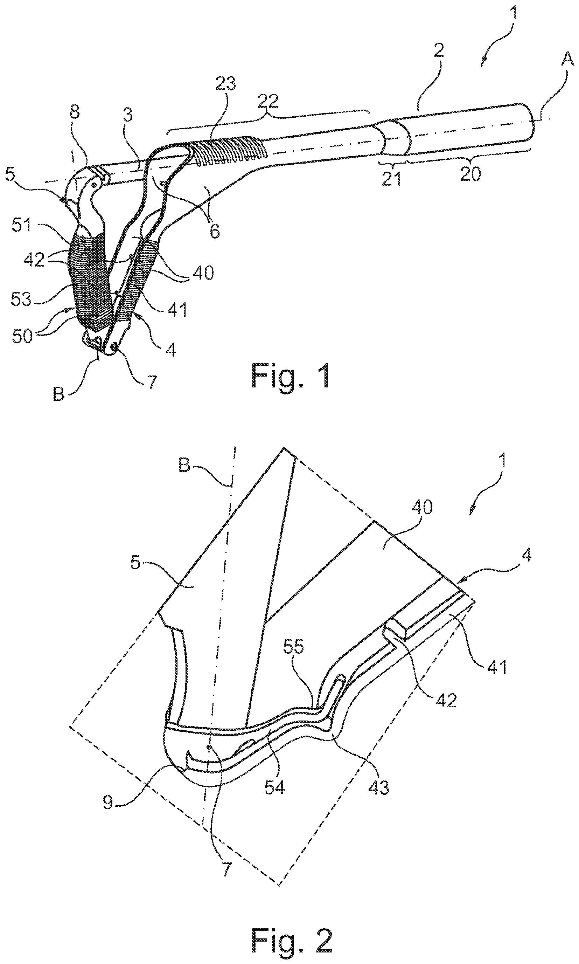

En référence aux figures 1 et 2, le dispositif d'introduction 1 d'un élément (non représenté) dans un corps (non représenté) selon l'invention comporte un tube de guidage 2, une tige de poussée 3, une poignée fixe 4 et une poignée mobile 5.

Le tube de guidage 2 est creux et a par exemple une section circulaire, ovoïdale ou toute autre section adaptée. Dans cet exemple, le tube de guidage 2 comporte trois portions distinctes à savoir une portion distale 20 et une portion proximale 22 raccordée par une portion intermédiaire 21. La portion distale 20 est apte à recevoir l'élément à introduire, elle a des dimensions intérieures supérieures à celles de la portion proximale 22 qui sert de guidage longitudinal à la tige de poussée 3 décrite plus loin. Ainsi, l'élément ne risque pas d'être placé trop en arrière dans le tube de guidage 2. Dans cet exemple, les portions distale 20 et proximale 22 sont sensiblement circulaires et la portion intermédiaire 21 sensiblement conique. Le tube de guidage 2 est prolongé par la poignée fixe 4 s'étendant dans le plan médian du dispositif d'introduction 1 en formant un angle avec l'axe longitudinal A du tube de guidage 1. Le tube de guidage 2 et la poignée fixe 4 forment ainsi une pièce monobloc permettant de réduire le nombre de pièce à assembler pour former le dispositif d'introduction 1. Le tube de guidage 2 et la poignée fixe 4 sont raccordés par des ailes de renfort 6 sensiblement parallèles entre-elles, permettant de former une zone de renfort rigidifiant l'ensemble. La poignée fixe 4 comporte deux flancs 40 reliés par une paroi dorsale 41 délimitant un logement, entièrement ouvert frontalement, apte à recevoir la poignée mobile 5 décrite plus loin. La paroi dorsale 41 peut être renforcée par des nervures 42. La poignée mobile 4 a ainsi une section en U. Les flancs 40, la paroi dorsale 41 ainsi que les ailes de renfort 6 sont prévus dans le prolongement direct de la paroi du tube de guidage 2 qui les forme en s'ouvrant, sans surépaisseur de paroi. L'extrémité libre de la portion distale 20 du tube de guidage 2 peut comporter une ouverture (non représentée) s'étendant longitudinalement vers la poignée fixe 4. Cette ouverture autorise le passage d'un lien de retenue (non représenté) attaché à l'élément et dont l'extrémité doit rester accessible lors de la mise en place de l'élément dans le corps. L'extrémité du lien de retenue peut ainsi être maintenue à l'extérieur du corps de l'animal. Ce lien de retenue permet ainsi, si nécessaire, d'extraire facilement l'élément par simple traction sur l'extrémité du lien de retenue accessible à l'extérieur de l'animal. La plus grande partie de la surface extérieure du tube de guidage 2 est prévue lisse avec une rugosité fine facilitant l'introduction de la portion distale 20 dans l'animal. La partie supérieure de la portion proximale 22 du tube de guidage 2 peut être pourvue de crans 23 facilitant la préhension et la manipulation du dispositif d'introduction 1. La paroi de l'extrémité libre de la poignée fixe 4 forme un bossage 43 apte à servir d'appui à une languette ressort 54 décrite plus loin. Le bossage 43 forme une excroissance extérieure visible sur la paroi dorsale 41 de la poignée fixe 4.

La tige de poussée 3 a des dimensions extérieures et une section lui permettant de coulisser librement dans le tube de guidage 2 dans lequel elle est en partie logée et mobile entre :
- une position proximale dans laquelle elle n'occupe pas la portion distale 20 du tube de guidage 2, la tige de poussée 3 restant néanmoins engagée dans la portion proximale 23 du tube de guidage 2, l'élément pouvant alors être logé dans la portion distale 20 du tube de guidage 2, et
- une position distale dans laquelle elle traverse la portion proximale 22 et occupe au moins en partie la portion distale 20 du tube de guidage 2, l'élément ayant ainsi été expulsé du tube de guidage 2 entre les positions proximale et distale de la tige de poussée 3. La tige de poussée 3 est par exemple formée d'un tube plein. Elle peut également être formée d'un tube creux, on prévoit dans ce cas qu'elle ait une extrémité distale obturée permettant de pousser l'élément en dehors du tube de guidage 2. L'extrémité de la tige de poussée 3 située à l'extérieur du tube de guidage 2 est couplée, par une première liaison pivot 7, à une poignée mobile 5 s'étendant dans le plan frontal du dispositif d'introduction 1. La poignée mobile 5 a une section en U définissant des flancs 50 et une paroi frontale 51 orientée à l'opposé de la poignée fixe 4. La poignée mobile 5 peut avoir toute autre section adaptée, par exemple une section pleine. Dans cet exemple, la surface extérieure de la poignée mobile 5 est pourvue de crans 53 facilitant sa manipulation et la préhension du dispositif d'introduction 1. Les flancs 41, 51 de la poignée fixe 4 et de la poignée mobile 5 sont dimensionnés de sorte que la poignée mobile 5 puisse être reçue, en tout ou partie, dans le logement de la poignée fixe 4. La première liaison pivot 7 est formée par des premiers tenons s'étendant latéralement de l'extrémité proximale de la tige de poussée 3 placée et reçus dans des premiers orifices prévus dans les flancs 50 d'une première extrémité de la poignée mobile 5. L'assemblage de la première liaison pivot 7 se fait par déformation élastique des flancs 50 de la poignée mobile 5. La première liaison pivot 7 peut bien entendue être réalisée de toute autre manière adaptée, les tenons étant par exemple prévus sur la poignée mobile et le ou les orifices dans la tige de poussée. La seconde extrémité de la poignée mobile 5 est couplée par une seconde liaison pivot 8 à l'extrémité libre de la poignée fixe 4. La seconde liaison pivot 8 est formée par des seconds tenons s'étendant latéralement des flancs 50 de la seconde extrémité de la poignée mobile 5 et reçus dans des seconds orifices prévus dans les flancs 40 de l'extrémité libre de la poignée fixe 4. L'assemblage de la seconde liaison pivot 8 se fait par déformation élastique des flancs 40 de la poignée fixe 4. La seconde liaison pivot peut bien entendue être réalisée de toute autre manière adaptée, les tenons étant par exemple prévus sur la poignée mobile et le ou les orifices dans la poignée mobile. Les première et seconde liaisons pivot 7, 8 faisant partie intégrante des pièces qu'elles servent, le nombre de pièce à assembler pour former le dispositif d'introduction 1 est limité d'autant. Les poignées mobile 5 et fixe 4 sont ainsi mobiles l'une par rapport à l'autre afin d'autoriser leurs fermeture et ouverture pour déplacer la tige de poussée 3 entre ses positions proximale et distale.

La seconde extrémité de la poignée mobile 5 est prolongée par une languette ressort 54 définissant un élément de rappel élastique et s'étendant sensiblement perpendiculairement à l'axe longitudinal B de la poignée mobile 5. La languette ressort 54 et la poignée mobile 5 forment ainsi une pièce monobloc permettant de réduire le nombre de pièce à assembler pour former le dispositif d'introduction 1 et de supprimer le risque de perdre la languette ressort 54 si elle devait être une pièce indépendante. La languette ressort 54 comporte deux portions angulairement distinctes définissant entre-elles un coude 55 apte à venir en appui contre le bossage 43 bloquant ainsi le coude 55. Les poignées fixe 4 et mobile 5 sont ainsi séparées par la languette ressort 54 qui tend à les ramener dans la position ouverte. Comme illustré sur la figure 2, les poignées fixe 4 et mobile 5 comportent respectivement des formes complémentaires 9, aptes à venir en butée entre-elles pour délimiter l'ouverture maximale entre les poignées fixe 4 et mobile 5 dans la position ouverte.

Selon un autre mode de réalisation non représenté, la poignée fixe est prévue couplée à la tige de poussée et la poignée mobile couplée au tube de guidage. De même, le tube de guidage peut avoir toute autre forme adaptée, par exemple coudée. Dans ce cas, la tige de poussée sera prévue flexible afin de s'adapter à ce coude. Selon encore un autre mode de réalisation non représenté, la languette ressort est couplée à la poignée fixe et en appui contre la poignée mobile.

L'utilisation du dispositif d'introduction 1 est décrite ci-après. L'élément à introduire dans le corps de l'animal est inséré manuellement dans la portion distale 20 du tube de guidage 2. L'utilisateur saisi le dispositif d'introduction 1 par ses poignées fixe 4 et mobile 5 et introduit la portion distale 20 dans le corps de l'animal. Lorsque l'élément est pourvu d'un lien de retenue, ce lien de retenue est passé au travers de l'ouverture prévue dans la portion distale 20 et l'utilisateur en maintien l'extrémité en dehors du corps de l'animal pendant l'introduction de la portion distale 20 du tube de guidage 2. Une fois la portion distale 20 en position dans le corps de l'animal, l'utilisateur déplace la poignée mobile 5 de sa position ouverte à sa position fermée vers la poignée fixe 4 pour déplacer la tige de poussée 3 de sa position proximale à sa position distale. L'élément est ainsi poussé, expulsé du tube de guidage 2 pour être laissé en place à l'intérieur du corps de l'animal. L'utilisateur peut retirer la portion distale 20 du corps de l'animal en veillant à ne pas entrainer le lien de retenue, puis relâche la poignée mobile 5. La languette ressort 54 ramène alors la poignée mobile 5 dans sa position ouverte et la tige de poussée 3 dans sa position proximale. Le dispositif d'introduction 1 peut être réutilisé immédiatement, après simple rechargement, dans le tube de guidage 2, d'un nouvel élément à introduire.

De manière générale, le dispositif d'introduction 1 selon l'invention permet d'introduire à l'intérieur d'un corps, par exemple celui d'un animal, un élément tel que par exemple un médicament, un traceur ou tout autre élément adapté. Le dispositif d'introduction 1 décrit est en particulier adapté pour introduire un diffuseur de produit actif dans le vagin d'un bovin afin de contrôler et/ou de réguler l'activité hormonale du bovin. Le dispositif d'introduction 1 selon l'invention est simple à fabriquer et assembler puisqu'il ne comporte que trois pièces à assembler, chacune étant monobloc et ayant une géométrie simple, à savoir :
- une première pièce comportant la tige de poussée 3 pourvue d'une partie de la première liaison pivot 7,
- une deuxième pièce comportant le tube de guidage 2 prolongé par la poignée fixe 4 pourvue d'une partie de la seconde liaison pivot 8,
- une troisième pièce comportant la poignée mobile 5 pourvue d'une partie de la première liaison pivot 7 et d'une partie de la seconde liaison pivot 8.
Le dispositif d'introduction 1 selon l'invention est facile à utiliser et à entretenir. En effet, l'élément de rappel élastique, dans l'exemple décrit la languette ressort 54, est dissimulé dans la poignée fixe 4, il ne risque ainsi pas d'être accroché de l'extérieur. Il peut de plus être facilement nettoyé.

## Revendications

1. Dispositif d'introduction (1) d'éléments dans un corps, comportant un tube de guidage (2) creux apte à recevoir au moins un élément à introduire, une tige de poussée (3) prévue coulissante dans ledit tube de guidage (2) entre une position proximale dans laquelle elle libère au moins en partie la portion distale (20) dudit tube de guidage (2) et une position distale dans laquelle elle occupe au moins en partie ladite portion distale (20) pour expulser ledit élément dudit tube de guidage (2), une poignée fixe (4) et une poignée mobile (5) couplées l'une audit tube de guidage (2), l'autre à ladite tige de poussée (3), ladite poignée mobile (5) étant couplée à ladite poignée fixe (4) par une première liaison pivot (7) et à ladite tige de poussée (3) par une seconde liaison pivot (8), lesdites première et seconde liaisons pivot (7, 8) autorisant la fermeture et l'ouverture desdites poignées mobile (5) et fixe (4) et le déplacement de ladite lige de poussée (3) entre ses positions proximale et distale, lesdites poignées mobile (5) et fixe (4) étant séparées par au moins un élément de rappel élastique (54) tendant à les ramener dans ladite position ouverte, ledit élément de rappel élastique étant formé par une languette ressort (54) prolongeant au moins une desdites poignées mobile (5), fixe (4), avec laquelle elle forme une pièce monobloc, l'extrémité libre de ladite languette ressort (54) prenant appui respectivement contre ladite poignée fixe (4), mobile (5), **caractérisé en ce que** ladite languette ressort (54) comporte au moins deux portions angulairement distinctes définissant entre-elles au moins un coude (55), **en ce que** l'une desdites poignée fixe (4), poignée mobile (5), comporte au moins un bossage (43) apte a recevoir ledit coude (55) pour bloquer la position de ladite languette ressort (54) par rapport respectivement à ladite poignée fixe (4), poignée mobile (5).

2. Dispositif d'introduction (1) selon la revendication précédente, **caractérisé en ce que** l'une au moins desdites première, seconde liaisons pivot (7, 8) comporte au moins un tenon et au moins une zone de guidage dudit tenon, prévus respectivement l'un sur ladite poignée mobile (5), l'autre sur l'une desdites tige de poussée (3), poignée fixe (5), et définissant respectivement avec chacune une pièce monobloc.

3. Dispositif d'introduction (1) selon l'une quelconque des revendications précédentes, **caractérisé en ce que** ladite poignée fixe (4) et ladite poignée mobile (5) comportent respectivement des formes complémentaires (8) aptes à venir en butée entre-elles pour délimiter une position ouverte maximale.

4. Dispositif d'introduction (1) selon l'une quelconque des revendications précédentes, **caractérisé en ce que** ladite poignée mobile (5) est couplée à ladite tige de poussée (3), **en ce que** ladite poignée fixe (5) est formée par le prolongement direct de la paroi dudit tube de guidage (2) et qu'elle comporte deux flancs (40) reliés par une paroi dorsale (41) délimitant un logement entièrement ouvert frontalement apte a recevoir ladite poignée mobile (5) au moins dans sa position fermée.

## Patentansprüche

1. Vorrichtung (1) zum Einführen von Elementen in einen Körper, umfassend ein hohles Führungsrohr (2), das geeignet ist, mindestens ein einzuführendes Element aufzunehmen, eine Schubstange (3), die in dem Führungsrohr (2) gleitend zwischen einer proximalen Position, in der diese mindestens einen Teil des distalen Abschnitts (20) des Führungsrohrs (2) freigibt, und einer distalen Position, in der diese mindestens teilweise den distalen Abschnitt (20) einnimmt, um das Element aus dem Führungsrohr (2) auszustoßen, vorgesehen ist, einen festen Griff (4) und einen bewegbaren Griff (5), wobei der eine mit dem Führungsrohr (2), und der andere mit der Schubstange (3) gekuppelt ist, wobei der bewegbare Griff (5) mit dem festen Griff (4) durch eine erste Schwenkverbindung (7) und mit der Schubstange (3) durch eine zweite Schwenkverbindung (8) gekoppelt ist, wobei die erste und zweite Schwenkverbindung (7, 8) das Schließen und das Öffnen des bewegbaren (5) und festen (4) Griffs und die Verschiebung der Schubstange (3) zwischen ihrer proximalen und distalen Position gestatten, wobei der bewegbare (5) und feste (4) Griff durch mindestens ein elastisches Rückstellelement (54) getrennt sind, das darauf hinwirkt, sie in die offene Position zurückzuführen, wobei das elastische Rückstellelement durch eine Federzunge (54) gebildet ist, die mindestens einen von dem mobilen (5) und festen (4) Griff verlängert, mit dem sie einstückig gebildet ist, wobei das freie Ende der Federzunge (54) jeweils gegen den festen (4) und bewegbaren (5) Griff anliegt,
**dadurch gekennzeichnet, dass** die Federzunge (54) mindestens zwei winkelmäßig verschiedene Abschnitte umfasst, die zwischen einander mindestens einen Bogen (55) definieren, dadurch, dass einer von dem festen Griff (4) und dem bewegbaren Griff (4) mindestens einen Vorsprung (43) aufweist, der geeignet ist, den Bogen (55) aufzunehmen, um die Position der Federzunge (54) jeweils relativ zu dem festen Griff (4) und dem bewegbaren Griff (5) zu blockieren.

2. Vorrichtung (1) zum Einführen nach dem vorhergehenden Anspruch, **dadurch gekennzeichnet, dass** mindestens eine der ersten und zweiten Schwenkverbindungen (7, 8) mindestens einen Zapfen und mindestens eine Führungszone des Zapfens umfasst, wobei jeweils eines an dem bewegbaren Griff (5) und das andere an einem von der Schubstange (3) und dem festen Griff (5) bereitgestellt ist und jeweils mit jedem einstückig ausgebildet ist.

3. Vorrichtung (1) zum Einführen nach einem der vorhergehenden Ansprüche, **dadurch gekennzeichnet, dass** der feste Griff (4) und der bewegbare Griff (5) jeweils komplementäre Formen (8) aufweisen, die geeignet sind, aneinander anzuliegen, um eine maximale offene Position zu begrenzen.

4. Vorrichtung (1) zum Einführen nach einem der vorhergehenden Ansprüche, **dadurch gekennzeichnet, dass** der bewegbare Griff (5) mit der Schubstange (3) gekoppelt ist, dadurch, dass der feste Griff (5) durch die direkte Verlängerung der Wand des Führungsrohrs (2) gebildet ist, und dass dieser zwei Flanken (40) umfasst, welche durch eine dorsale Wand (41) verbunden sind, die eine vollständig frontal offene Aufnahme begrenzt, welche geeignet ist, den bewegbaren Griff (5) mindestens in seiner geschlossenen Position aufzunehmen.

## Claims

1. Device (1) for introducing elements into a body, comprising a hollow guide tube (2) suitable for receiving at least one element to be introduced, a push rod (3) being slidable in said guide tube (2) between a proximal position wherein it releases at least partially the distal portion (20) of said guide tube (2) and a distal position wherein it occupies at least partially said distal portion (20) in order to expel said element from said guide tube (2), a stationary grip (4) and a movable grip (5), one coupled to said guide tube (2), the other coupled to said push rod (3), said movable grip (5) being coupled to said stationary grip (4) by a first pivoting link (7) and to said push rod (3) by a second pivoting link (8), said first pivoting link and second pivoting link (7, 8) allowing the closure and opening of said movable grip (5) and stationary grip (4) and the movement of said push rod (3) between its proximal and distal positions, said movable grip (5) and stationary grip (4) being separated by at least one resilient return element (54) tending to return them to said open position, said resilient return element being formed by a spring tab (54) extending at least one of said items: movable grip (5), stationary grip (4), with which it forms a unitary part, the free end of said spring tab (54) bearing against said stationary grip (4), movable grip (5), respectively,
**characterised in that** said spring tab (54) comprises at least two angularly separate portions defining between themselves at least one bend (55), **in that** one of said items:
stationary grip (4), movable grip (5), comprises at least one boss (43) suitable for receiving said bend (55) in order to lock the position of said spring tab (54) relative to said stationary grip (4), movable grip (5), respectively.

2. Introducing device (1) according to the preceding claim, **characterised in that** at least one of said items: first pivoting link, second pivoting link (7, 8) comprises at least one lug and at least one area for guiding said lug, one provided on said movable grip (5), the other provided on one of said items: push rod (3), stationary grip (5), respectively, and defining a unitary part with each one, respectively.

3. Introducing device (1) according to any one of the preceding claims, **characterised in that** said stationary grip (4) and said movable grip (5) comprise complementary shapes (8), respectively, suitable for abutting against each other in order to define a maximum open position.

4. Introducing device (1) according to any one of the preceding claims, **characterised in that** said movable grip (5) is coupled to said push rod (3), **in that** said stationary grip (5) is formed by the direct extension of the wall of said guide tube (2) and **in that** it comprises two flanks (40) connected by a rear wall (41) defining a recess fully open at the front, suitable for receiving said movable grip (5) at least in its closed position.
